Europäisches Patentamt

European Patent Office

Office européen des brevets

(19)

(11) Veröffentlichungsnummer: **0 028 864**
**B1**

(12) **EUROPÄISCHE PATENTSCHRIFT**

(45) Veröffentlichungstag der Patentschrift:
19.03.86

(51) Int. Cl.⁴: **A 61 B 6/00**

(21) Anmeldenummer: **80201057.9**

(22) Anmeldetag: **07.11.80**

(54) Verfahren zur Darstellung artefaktarmer Schichtbilder eines dreidimensionalen Objektes.

| | |
|---|---|
| (30) Priorität: **10.11.79 DE 2945536** | (73) Patentinhaber: **Philips Patentverwaltung GmbH, Bilistrasse 80, D-2000 Hamburg 28 (DE)** |
| | (84) Benannte Vertragsstaaten: **DE** |
| (43) Veröffentlichungstag der Anmeldung: **20.05.81 Patentblatt 81/20** | (73) Patentinhaber: **N.V. Philips' Gloeilampenfabrieken, Groenewoudseweg 1, NL-5621 BA Eindhoven (NL)** |
| (45) Bekanntmachung des Hinweises auf die Patenterteilung: **19.03.86 Patentblatt 86/12** | (84) Benannte Vertragsstaaten: **FR GB IT SE** |
| (84) Benannte Vertragsstaaten: **DE FR GB IT SE** | (72) Erfinder: **Klotz, Erhard, Seekamp 110, D-2083 Halstenbek (DE)** Erfinder: **Linde, Rolf, Kamperrege 21, D-2081 Haseldorf (DE)** Erfinder: **Tiemens, Ulf, Hauptstrasse 37B, D-2081 Prisdorf (DE)** Erfinder: **Weiss, Hermann, Mester Brooksweg 2, D-2000 Hamburg 65 (DE)** |
| (56) Entgegenhaltungen: DE - A - 1 950 966 DE - A - 2 461 877 DE - A - 2 646 696 DE - A - 2 722 141 DE - A - 2 746 035 DE - B - 2 414 322 | (74) Vertreter: **Hartmann, Heinrich, Dipl.-Ing. et al, Philips Patentverwaltung GmbH Bilistrasse 80 Postfach 10 51 49, D-2000 Hamburg 28 (DE)** |

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Darstellung von Schichtbildern eines dreidimensionalen Objektes, das von einer Vielzahl von in einer Ebene liegenden Strahlenquellenpositionen aus zur Aufnahme wenigstens eines aus einzelnen Perspektivbildern bestehenden kodierten Bildes des Objektes durchstrahlt wird, wobei aus dem kodierten Bild, dessen Perspektivbilder mit einer Abbildungsmatrix vervielfacht abgebildet werden, deren Abbildungselemente entsprechend der ebenen Verteilung der Strahlenquellenpositionen verteilt sind, infolge der Überlagerung der perspektivbilder Schichtbilder des Objektes auf einer lichtempfindlichen Schicht erzeugt werden.

Ein derartiges Verfahren ist bereits aus den DE-OS 24 14 322 und 27 46 035 bekannt. Hier wird ein Objekt mit einer Anzahl von in einer Strahlenquellenebene liegenden Röntgenröhren zur Aufnahme eines aus einzelnen Perspektivbildern bestehenden kodierten Bildes des Objektes durchstrahlt, wobei das kodierte Bild z.B. auf einem Film aufgenommen wird. Mit Hilfe einer Abbildungsmatrix, deren Abbildungselemente entsprechend der ebenen Verteilung der Strahlenquellen angeordnet sind, können dann aus dem kodierten Bild beliebige Schichtbilder des Objektes erzeugt werden. Die Bildqualität dieser Schichtbilder wird aber durch Artefakte erheblich beeinträchtigt, die in besonderem Maße bei sich stark überlagernden Perspektivbildern auftreten. Dies ist z.B. immer dann der Fall, wenn große Objektfelder aus einer großen Anzahl von Richtungen durchstrahlt werden.

Die Artefakte werden dabei häufig von Objektstrukturen verursacht, die zur Diagnose an sich gar nicht oder nur unwesentlich herangezogen werden. Bei Gefäßuntersuchungen, z.B. in der Angiographie, sind die relevanten Objektdetails die mit Kontrastmittel gefüllten Gefäße, während die umgebenden Knochenstrukturen oftmals nur von untergeordneter Bedeutung sind und z.B. nur zur Orientierung dienen. Diese Knochenstrukturen verursachen aber erhebliche Artefakte in den rekonstruierten Schichtbildern des Objektes.

Es ist bekannt, daß z.B. in der Angiographie unerwünschte Knochenstrukturen in einfachen Röntgenbildern (Schattenbildern) durch Subtraktionstechniken unterdrückt werden können (Ziedses des Plantes, Amsterdam 1961, Georg Thieme Verlag, Stuttgart). Es handelt sich hierbei jedoch nicht um Röntgenbilder, aus denen Schichtbilder des durchstrahlten Objektes rekonstruiert werden können.

Ferner ist aus der DE-OS 27 22 141 ein Verfahren bekannt, bei dem eine derartige Subtraktionstechnik auf Schichtbilder eines Objektes angewendet wird. Nachteilig bei diesem Verfahren ist jedoch, daß für jede darzustellende Schicht des Objektes zwei Schichtbilder erzeugt werden müssen, von denen das eine z.B. das normale Objekt und das andere das mit einem Kontrastmittel gefüllte Objekt darstellt. Beide Schichtbilder werden dann zur Darstellung des mit Kontrastmittel gefüllten Objektdetails voneinander subtrahiert. Zur Darstellung jeweils einer weiteren Schicht des Objektes müssen jeweils zwei neue Schichtbilder erzeugt werden, so daß die Untersuchungszeit des Objektes relativ lang ist.

Nachteilig bei dem obigen Verfahren ist ferner die nicht gleichzeitige Aufnahme aller zu einem kodierten Bild gehörenden Perspektivbilder, so daß Fehler in den Schichtbildern aufgrund der Bewegung des Objektes und aufgrund unterschiedlicher, sich z.B. zeitlich verändernder Kontrastphasen (z.B. fließendes Kontrastmittel) in den Objektdetails auftreten können.

Aufgabe der Erfindung ist es, ein Verfahren anzugeben, mit dessen Hilfe mit Kontrastmittel gefüllte Objektdetails durch artefaktarme Schichtbilder des Objektes dargestellt werden können.

Diese Aufgabe wird gemäß der Erfindung dadurch gelöst, daß zur Darstellung von mit einem Kontrastmittel füllbaren Objektdetails von dem Objekt jeweils kodierte Bilder in unterschiedlichen Kontrastphasen der Objektdetails hergestellt werden, und daß jeweils zwei unterschiedliche kodierte Bilder voneinander subtrahiert und überlagert werden.

Möchte man unnötige Objektstrukturen aus Mehrfachperspektivbildern (kodierten Bildern) eliminieren, so daß die bildverschlechternden Artefakte bei der Tomosynthese vermindert werden, so wird z.B. bei Gefäßuntersuchungen zuerst ein negatives Mehrfachperspektivbild (kodiertes Bild) in Form einer "Leeraufnahme" (Aufnahme ohne Kontrastmittel) aufgenommen. Danach wird das mit Kontrastmittel gefüllte Objektdetail als negatives Mehrfachperspektivbild (negatives kodiertes Kontrastbild) aufgezeichnet ("Füllungsaufnahme").

Die in diesen Bildern nicht notwendigen Objektdetails (z.B. Knochen), die bei Gefäßdarstellungen mit Hilfe der Tomosynthese nur störende Artefakte verursachen, können dann mit Hilfe der Mehrfachperspektiv-Leeraufnahme eliminiert werden. Dazu wird z.B. ein Positiv der Leeraufnahme hergestellt, das anschließend mit den negativen Mehrfachperspektiv-Füllungsaufnahmen jeweils überlagert wird. Bei unveränderter Lage des Patienten während der Aufnahmezeit können somit die störenden Bilddetails der gesamten Aufnahmeserie mit nur einer einzigen Leeraufnahme eliminiert werden, so daß die Artefakte in den Schichtbildern vermindert werden. Die Strahlenbelastung für den Patienten erhöht sich dabei nur um eine zusätzliche Aufnahme (Leeraufnahme), während die Diagnostizierbarkeit der Schichtbilder dadurch wesentlich verbessert werden kann. Gleichzeitig werden mit Hilfe der Leeraufnahme nicht nur Artefakte vermindert, die in der momentan dargestellten Schicht verlaufen, sondern es werden auch die Artefakte aus allen anderen Schichten vermindert.

Natürlich kann die Leeraufnahme auch durch eine andere geeignete Füllungsaufnahme ersetzt werden, so daß dann zwei Füllungsaufnahmen mit unterschiedlichen Kontrastphasen zur Schichtbilderzeugung verwendet werden. Unterschiedliche Kontrastphasen des Objektdetails können z.B. dann vorliegen, wenn ein Objektdetail einmal zur Hälfte und einmal ganz mit Kontrastmittel gefüllt ist. Durch Differenzbildung zweier Füllungsaufnahmen kann dann der Füllungsstand der Objektdetails in einem bestimmten Zeitintervall dargestellt werden.

Die Zeichnung stellt ein Ausführungsbeispiel der Erfindung dar.

Es zeigen:

Fig. 1 eine Vorrichtung zur Aufnahme der kodierten Bilder,

Fig. 2a einen Bildausschnitt einer Mehrfachperspektiv-Leeraufnahme (Negativ),

Fig. 2b ein Positiv der Mehrfachperspektiv-Leeraufnahme nach Fig. 2a,

Fig. 2c einen Bildausschnitt einer Mehrfachperspektiv-Füllungsaufnahme (Negativ),

Fig. 2d das Ergebnis einer Überlagerung der Bilder aus den Fig. 2b und c,

Fig. 3 einen Dekodieraufbau zur Rekonstruktion von Schichtbildern und

Fig. 4 einen Dekodieraufbau für zwei kodierte Bilder.

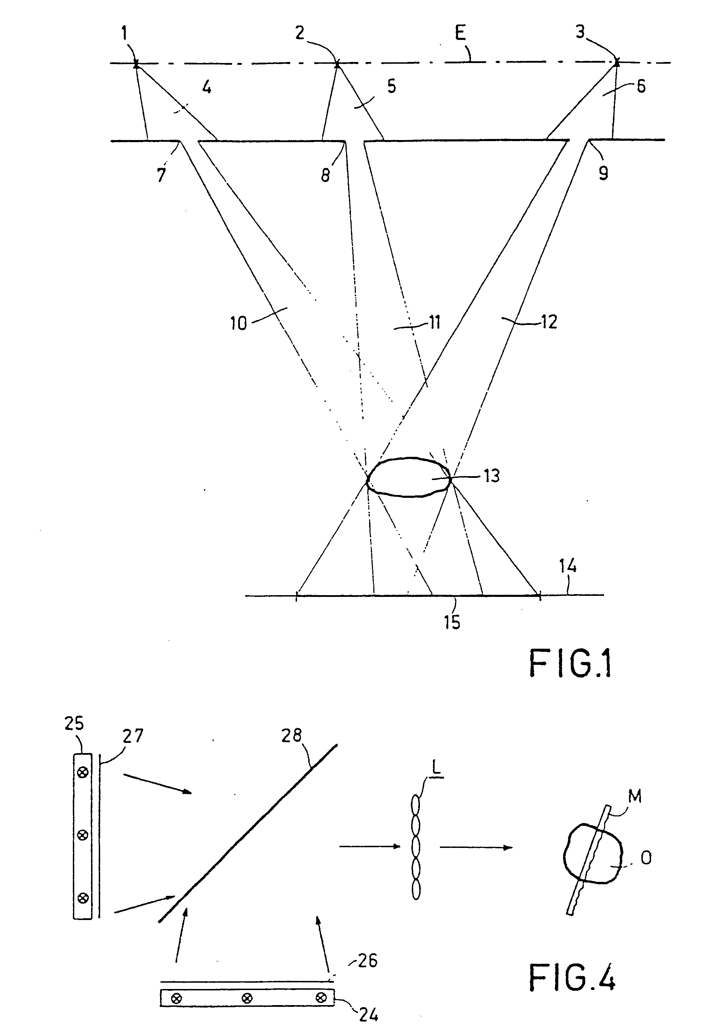

Die Fig. 1 zeigt schematisch eine Vorrichtung zur Aufnahme kodierter Bilder mit drei Röntgenröhren 1, 2 und 3, die in einer Strahlenquellenebene E angeordnet sind und deren Primärstrahlenbündel 4, 5, 6 durch Blendenöffnungen 7, 8, 9 soweit ausgeblendet sind, daß die restlichen Primärstrahlen 10, 11, 12 ein zu untersuchendes Objekt 13 durchstrahlen und auf einer lichtempfindlichen Schicht 14, z.B. einem Film, ein aus einzelnen Perspektivbildern bestehendes Mehrfachperspektivbild 15 (kodiertes Bild) erzeugen. Die Röntgenröhren 1, 2 und 3 können dabei gleichzeitig geblitzt werden.

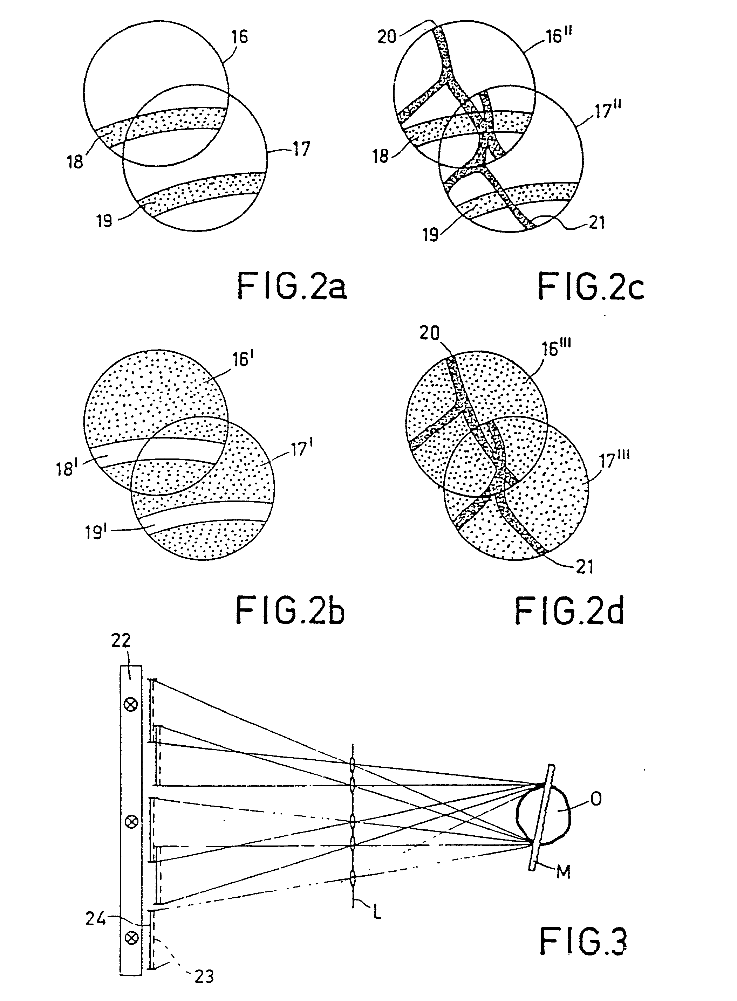

Die Fig. 2a zeigt eine, Bildausschnitt einer "Mehrfachperspektiv-Leeraufnahme"(Negativ) mit zwei Perspektivbildern 16 und 17. In diesen Bildern ist nur eine Knochenstruktur 18 und 19 des Objektes 13 enthalten. Ein bei der Leeraufnahme noch nicht gefülltes Gefäß-System wird in dieser Röntgenaufnahme noch nicht sichtbar.

Die Fig. 2b zeigt die in Fig. 2a dargestellten Perspektivbilder 16, 17 in umkopierter Form als Positiv (16', 17'). Dieser Umkopierungsprozeß kann nach bekannten Verfahren, z.B. photographisch oder fernsehtechnisch, erfolgen.

Die Fig. 2c zeigt die mit Fig. 2a identischen Perspektivbilder 16'', 17'', jedoch jetzt mit Kontrastmittelfüllung des Gefäßes. in dem Bildausschnitt sind wieder die Knochenstrukturen 18 und 19 und zusätzlich das Gefäß-System 20 und 21 dargestellt (Objektdetails).

In Fig. 2d ist das Ergebnis einer Überlagerung der Bildausschnitte aus Fig. 2b und Fig. 2c dargestellt. In diesen Perspektivbildern 16''' und 17''' verschwinden die Knochenstrukturen 18 und 19 und es bleibt nur das gefüllte Gefäß-System 20 und 21 übrig, d.h. die Differenz der Bildinformation. Genaue Schwärzungsanteile, hervorgerufen durch die Überlagerung der Bilder, wurden in den Fig. 2a-c nicht berücksichtigt. Aus derartigen Perspektivbildern 16''', 17''' können dann artefaktarme Schichtbilder des Objektes rekonstruiert werden.

Die Fig. 3 zeigt einen bekannten Dekodieraufbau aus der Tomosynthese, mit dem die Mehrfachperspektivbilder ausgewertet werden. Ein Lichtkasten 22 beleuchtet die Mehrfachperspektivbilder 23 und 24. Das Mehrfachperspektivbild 23 besreht z.B. aus den Teilbildern der Mehrfachperspektiv-Füllungsaufnahme aus Fig. 2c, während das Mehrfachperspektivbild 24 aus der photographisch umkopierten Mehrfachperspektiv-Leeraufnahme (gestrichelt gezeichnet) der Fig. 2b besteht.

Diese beide, Aufnahmen, bei denen es sich jeweils auch um photographische Kopien handel, kann, sind zur Deckung gebracht worden. Mit Hilfe der Linsenmatrix L wird das quasi dreidimensionale Objekt 0 rekonstruiert. Die Verteilung der Linsen innerhalb der Linsenmatrix entspricht dabei der ebenen Verteilung der Röntgenröhren. Auf einer Mattscheibe M, die im reelle, Bild des rekonstruierten Objektes 0 beliebig bewegt werden kann, können unterschiedlich gelagerte Schichten des Objektes dargestellt werden. Die Schichtbilder sind frei von den störenden Artefakten, da die Knochenstrukturen mit Hilfe der Leeraufnahme eliminiert wurden.

Die artefaktfreie Darstellung gilt dabei für den gesamten rekonstruierten Objektbereich, in dem auch sog. Schrägschichten des Objektes 0 artefaktfrei erzeugt werden können.

In vielen Fällen ist es vorteilhaft, die Knochenstrukturen nicht vollständig zu eliminieren, weil so die Übersicht über die Lage der mit Kontrastmittel gefüllten Gefäße nicht vollständig verloren geht. In der Fig. 4 ist eine Vorrichtung dargestellt, mit der die Knochenstrukturen kontinuierlich eliminiert werden können. Diese Vorrichtung besitzt zwei Lichtkästen 24, 25, vor denen jeweils eine Mehrfachperspektiv-Füllungsaufnahme 26 als Negativ oder eine Mehrfachperspektiv-Leeraufnahme 27 als Positiv liegen. Beide Mehrfachperspektiv-Aufnahmen 26, 27 werden mit Hilfe eines halbdurchlässigen Spiegels 28 überlagert und mittels der Linsenmatrix L dekodiert, so daß artefaktarme Schichtbilder auf der Mattscheibe M im rekonstruierten Objekt 0 entstehen. Für den Fall, daß beide Mehrfachperspektiv-Aufnahmen 26, 27 gleich hell beleuchtet sind, werden die Artefakte maximal reduziert. Bei unterschiedlich starker Beleuchtung treten die Knochenstrukturen 18, 19 (Fig. 2) und damit die Artefakte zwar wieder auf, jedoch ist anhand der Knochenstrukturen 18, 19 eine Orientierung leichter möglich. Aus diesem Grunde kann wenigstens ein Lichtkasten 24, 25 in seiner Helligkeit verändert werden.

## Patentansprüche:

1. Verfahren zur Darstellung von Schichtbildern eines dreidimensionalen Objektes, das von einer Vielzahl von in einer Ebene liegenden Strahlenquellenpositionen aus zur Aufnahme wenigstens eines aus einzelnen Perspektivbildern bestehenden kodierten Bildes des Objektes durchstrahlt wird, wobei aus dem kodierten Bild, dessen Perspektivbilder mit einer Abbildungsmatrix vervielfacht abgebildet werden, deren Abbildungselemente entsprechend der ebenen Verteilung der Strahlenquellenpositionen verteilt sind, infolge der Überlagerung der Perspektivbilder Schichtbilder des Objektes auf einer lichtempfindlichen Schicht erzeugt werden, dadurch gekennzeichnet, daß zur Darstellung von mit einem Kontrastmittel füllbaren Objektdetails (20, 21) von dem Objekt jeweils kodierte Bilder (16, 17; 16", 17") in unterschiedlichen Kontrastphasen der Objektdetails hergestellt werden, und daß jeweils zwei unterschiedliche kodierte Bilder voneinander subtrahiert und überlagert werden.

2. Verfahren nach Anspruch 1, dadurch gekennzeichnet, daß zur Subtraktion von jeweils zwei negativen kodierten Bildern (16, 17; 16", 17") eines zur Erzeugung eines positiven kodierten Bildes (16', 17') umkopiert wird, und daß das negative und das eine andere Kontrastphase darstellende positive kodierte Bild überlagert werden.

3. Verfahren nach Anspruch 1 und 2, dadurch gekennzeichnet, daß die kodierten Bilder jeweils auf einen Film aufgenommen werden, und daß die Umkopierung auf photographischem Wege und die Überlagerung auf optischem Wege erfolgt.

4. Verfahren nach Anspruch 1 und 2, daß die kodierten Bilder jeweils elektronisch aufgezeichnet werden, und daß die Umkopierung und Üerlagerung auf elektronischem wege erfolgt.

5. Verfahren nach einem oder mehreren der Ansprüche 1 bis 4, dadurch gekennzeichnet, daß die kodierten Bilder mit unterschiedlich starker und kontinuierlich veränderbarer Helligkeit voneinander subtrahiert werden.

6. Vorrichtung zur Durchführung des Verfahrens nach einem oder mehreren der Ansprüche 1 bis 3 und 5, mit einer Beleuchtungsvorrichtung (24) zur Durchstrahlung eines kodierten Bildes und einer parallel zum kodierten Bild angeordneten, einzelne Linsen enthaltenden Linsenmatrix zur Überlagerung der Perspektivbilder, wobei die Verteilung der Linsen der ebenen Verteilung der Strahlenquellenpositionen entspricht, und mit einer im Überlagerungsbereich der Perspektivbilder des kodierten Bildes beliebig anordbaren lichtempfindlichen Schicht zur Abbildung von Schichtbildern, dadurch gekennzeichnet, daß im Strahlengang zwischen dem kodierten Bild (27) und der Linsenmatrix (L) ein halbdurchlässiger Spiegel (28) zur Überlagerung eines weiteren kodierten Bildes (26) mit dem ursprünglichen kodierten Bild (27) angeordnet ist, und mit einer weiteren Beleuchtungsvorrichrung (25) zur Durchstrahlung des kodierten Kontrastbildes.

7. Vorrichrung nach Anspruch 6, dadurch gekennzeichnet, daß wenigstens eine Beleuchtungsvorrichtung in ihrer Helligkeit veränderbar ist.

## Claims:

1. A process for displaying tomographic images of a threedimensional object which is irradiated from a number of radiation source positions which are situated in one plane in order to record at least one coded image of the object which consists of separate perspective images, tomographic images of the object being formed from the coded image whose perspective images are imaged in multiple form by means of an imaging matrix whose imaging elements are distributed in accordance with the flat distribution of the radiation source positions, by superpositions of the perspective images on a photosensitive layer, characterized in that for the display of object details (20, 21) which can be filled with a contrast medium, coded images (16, 17; 16",17") of the object are formed in different contrast phases of the object details, each time two different coded images being mutually subtracted and superposed.

2. A method as claimed in Claim 1, characterized in that for the subtraction of each time two negative coded images (16, 17; 16", 17"), one image is reversed in order to form a positive coded image (16', 17'), the negative coded image and the positive coded image which represents a different contrast phase being superposed.

3. A method as claimed in Claims 1 and 2, characterized in that the coded images are recorded on a film, the reversal being realized photographically, whilst the superposition is optically performed.

4. A method as claimed in the Claims 1 and 2, characterized in that the coded images are electronically recorded, the reversal and superposition being electronically realized.

5. A method as claimed in one more of the Claims 1 to 4, characterized in that the coded images with a different and continuously adjustable brightness are subtracted from each other.

6. A device for performing the method claimed in one or more of the Claims 1 to 3 and 5, comprising an illumination device (24) for irradiating a coded image and lens matrix which is arranged parallel to the coded image and which comprises separate lenses for the superposition of the perspective images, the distribution of the lenses corresponding to the flat distribution of the radiation source positions, and also comprising a photosensitive layer for the imaging of tomographic images which can be situated at random in the superposition zone of the perspective images of the coded image characterized in that in the beam path between the coded image (27) and the lens matrix (L) there is arranged a semitransparent mirror (28) for the superposition of a further coded image (26) with the original coded image (27), there also being provided a further illumination device (25) for irradiation of the coded contrast image.

7. A device as claimed in Claim 6, characterized in that the brightness of at least one illumination device is adjustable.

## Revendications

1.- Procédé pour la representation d'images tomographiques d'un objet tridimensionnel, qui, à partir d'un grand nombre de positions de sources de rayonnement disposées dans un plan, est traversé par le rayonnement en vue de l'enregistrement d'au moins une image codée de l'objet formée d'images en perspective individuelles, étant entendu qu'au départ de l'image codée, dont les images en perspective sont projetées en multiple au moyen d'une matrice de projection dont les éléments de projection sont répartis selon la répartition dans un plan des positions des sources de rayonnement, en conséquence de la superposition des images en perspective, des images tomographiques de l'objet sont produites sur une couche photosensible, caractérisé en ce que pour la représentation de details de l'objet (20, 21) pouvant être rempli d'un agent de contraste, des images codées (16, 17; 16'', 17'') de l'objet dans des phases de contraste differentes des details de cet objet sont produites et deux images codées differentes sont chaque fois soustraites l'une de l'autre et superposées.

2.- Procede suivant la revendication 1, caractérisé en ce que pour la soustraction de chaque fois deux images codées negatives (16, 17; 16'', 17''), l'une de celles-ci est tirée Pour produire une image codée positive (16', 17') et l'image negative ainsi que l'image codée positive représentant une autre phase de contraste sont superposées.

3.- Procédé suivant les revendications 1 et 2, caractérisé en ce que les images codées sont chaque fois enregistrées sur un film et le tirage s'effectue Par voie photographique, tandis que la superposition s'effectue par voie optique.

4.- Procédé suivant les revendications 1 et 2, caractérisé en ce que les images codées sont chaque fois enregistrées electroniquement et le tirage ainsi que la superposition s'effectuent par voie électronique.

5.- Procédé suivant l'une quelconque des revendications 1 à 4, caractérisé en ce que les images codées sont soustraites l'une de l'autre avec un éclairage différent et continuellement variable.

6.- Dispositif pour l'exécution du procédé suivant l'une quelconque des revendications 1 à 3 et 5, comportant un dispositif d'éclairage (24) pour éclairer une image codée et une matrice de lentilles contenant des lentilles individuelles disposée parallélement à l'image codée pour superposer les images en perspective, étant entendu que la répartition des lentilles correspond à la répartition dans un plan des positions de sources de rayonnement, et une couche photosensible destinée à la représentation des images tomographiques qui peut être placée à un endroit quelconque souhaité dans la zone de superposition des images en perspective de l'image codée, caractérisé en ce que dans le trajet des rayons entre l'image codée (27) et la matrice de lentilles L est disposé un miroir semi-transparent (28), destiné à superposer une autre image codée (26) sur l'image codée initiale (27), et un autre dispositif d'éclairage (25) est prévu pour éclairer l'image contrastée codée en transmission.

7.- Dispositif suivant la revendication 6, caractérisé en ce que la puissance d'éclairage d'au moins un dispositif d'éclairage est variable.

FIG.1

FIG.4

FIG.2a

FIG.2c

FIG.2b

FIG.2d

FIG.3

3